# NEW EUROPEAN PATENT SPECIFICATION

(11) **EP 0 329 879 B2**
(45) Date of publication and mention of the opposition decision: **16.04.1997**
(45) Mention of the grant of the patent: 01.09.1993
(21) Application number: 88301678.4
(22) Date of filing: 26.02.1988
(51) Int. Cl.: A61K 31/195

(54) **Amino acids useful in hepatic disorders**
Aminosäuren, nützlich bei Leberstörungen
Amino-acides utiles contre les troubles hépatiques

(30) Priority: 24.02.1988 JP 41368/88
(43) Date of publication of application: 30.08.1989
(73) Proprietor: Ajinomoto Co., Inc., Tokyo 104 (JP)
(72) Inventor: Masaki, Hisanori, Kawasaki-shi Kanagawa-ken (JP); Torii, Kunio, Kawasaki-shi Kanagawa-ken (JP); Suda, Tomio, Tachikawa-shi Tokyo (JP)
(74) Representative: Harrison, David Christopher

(56) References cited:
- FR-A- 2 217 002
- JP-A- 6 354 230
- US-A- 4 596 825
- J.E.F. REYNOLDS: "Martindale, the extra pharmacopoeia", 28th ed., 1982, The Pharmaceutical Press, London, GB; p. 48, ref. no. 572-b: "Alanine"
- DR. O.-A. NEUMÜLLER: "Römpps Chemie-Lexikon", 8th ed., vol. 2: "Cm-G", 1981, Franckh'sche Verlagshandlung, Stuttgart, DE; p. 1509, left-hand column: "Glutamin"
- Rote Liste, 1985 (33001)
- Garbin et al., MINERVA MEDICA, vol. 59, no. 80, 1968, pp. 4254-4260
- Fresenius: Infusionstherapie und Klinische Ernährung, 1983, pp. 336-338
- D. Perez-Sala et al.: Biochem.J., vol. 241, pp. 491-498
- J. Funovics et al.: Klin. Wochenschr., vol. 59, 1981, pp. 797-802
- J.M. Ravel et al.: The Journal of Biological Chemistry, 1955, pp. 497-501
- Pschyrembel Klinisches Wörterbuch, 1982, 254 Auflage, pp. 31, 662-663

## Description

This invention relates to drugs for use in connection with hepatic disorders.

The present invention provides: the use of L-alanine or a metabolic equivalent thereof and L-glutamine or a metabolic equivalent thereof in the manufacture of a pharmaceutical composition for the treatment or prophylaxis of an alcoholic hepatic disorder.

The L-glutamine may be accompanied by D-glutamine and/or the L-alanine may be accompanied by D-alanine.

The L-alanine and L-glutamine are optionally in forms which are metabolically equivalent thereto such as a salt, derivative or peptide.

Preferably, the L-alanine and L-glutamine are used in amounts such that the molar ratio of L-alanine to L-glutamine in the compositon is from 1:0.1 to 1:10; particularly preferably it is about 1:1. The invention therefore provides in one aspect a pharmaceutical composition for use in the therapy or prevention of hepatic disorders the molar ratio of L-alanine to L-glutamine in the composition being about 1:1.

In one embodiment L-alanine and L-glutamine or their metabolic equivalents are used in the manufacture of a two component composition, one component containing said L-alanine or derivative thereof , the second component containing said L-glutamine or derivative thereof.

In preferred embodiments the composition manufactured further comprises a pharmaceutically acceptable diluent, or carrier such that the composition is suitable for oral administration. The composition may, for example, be in the form of a powder, granules, tablet capsule, liquid or chewing gum. Preferably the composition is suitable for providing a dosage of 1 to 20g/person/day for example to a person of body weight 40 to 70 kg.

This invention relates to newly developed drugs or foods suitable for the therapy of patients with alcoholic hepatic disorders and for the prevention of such disorders.

The relatively excessive ingestion of alcoholic drinks over long time periods elicits various diseases, especially hepatic disorders and dysfunctions, such as fatty liver, alcoholic hepatitis, cirrhosis and so on, because the ethanol is catabolized specifically in the liver.

Total abstinence from alcoholic beverages should be recommended basically to patients with alcoholic hepatic disorders, who also receive therapy by drugs containing for example vitamins, phospholipids, glucocorticoids or insulin. In addition, drugs containing glucuronate or amino acid, for example, L-arginine hydrochloride, are available for use in these patients. Nonetheless, it is realized that these treatments and prescriptions often fail to achieve complete regression, because patients still indulge in drink occasionally despite the total abstinence strongly suggested by clinical medicine.

Recently, it became clear that some amino acids, for example, Ala were quite potent in enhancing the survival rate in mice, which received a lethal dose of ethanol. Especially, the treatment of Ala concurrently with L-ornithine was synergistically effective (Petition for Japanese patent, Showa 61-50917 US-A-4 596 825). We also recognized that rats displayed a preference for Ala as well as Gln under dietary ethanol loading over long periods (6 months or more), and that pretreatment with both compounds ameliorated both ethanol clearance from the blood and the recovery from the comatose state and the subsequent depression behaviourly following the ethanol loading. But it is very difficult from these facts to extrapolate whether therapy with Ala and Gln for the progressed state of alcoholic hepatic disorders would be effective or not. It is known that the dizziness after drink is closely related to the concentration of acetaldehyde in blood which as a metabolite of ethanol is elevated substantially after drink. Besides, the pretreatment with Ala, L-glutamate or aspartate decreased the toxicity of acetaldehyde as a catabolite of ethanol, but was not effective to suppress the serum enzymatic activities, being released from the damaged liver by the ethanol loading (Petition of Japanese patent. Showa 61-134313).

EP-B-0 087 750 discloses a dipeptide of ala-gln for parenteral nutrition of patients with liver insufficiency.

Minerva Medica 59,4254-4261(1968); O Garbin et la reports the results of a clinical trial in which glutamine was administered parenterally to a group of alcoholic patients. The paper includes a bibliography of references several of which relate to the use of glutamine in the treatment of alcoholism.

The therapy for patients with alcoholic hepatic disorders, induced by excessive ingestion of alcoholic beverages, has usually focused on ameliorating the degree of hepatic damage, mainly to the parenchymal cells, but complete regression is difficult to achieve in patients when the condition of the liver has become worse and chronic. In addition, it is very hard to bring back to normal the serum enzymatic activities elevated by alcoholic hepatic damage because patients still indulge in drink. Drugs for the treatment of hepatic damage and its prevention have been expected.

We inventors previously recognized that the plasma concentration of some L-amino acids, i.e., Ala and Gln, were reduced after ethanol loading, and these changes were suppressed by pretreatment with both compounds. This pretreatment is also effective to ameliorate alcohol-specific syndromes including hypoglycemia, retention of ethanol in the blood and the state of coma and depressed behaviour following excessive ingestion of alcoholic beverages. But there were insufficient data for Ala and Gln to establish the relationship with the degree of alcoholic hepatic disorders, such as serum enzymatic activities.

So, we tried to confirm the possibility of use as a drug and establish the optimal ratio of Ala and Gln on a molar basis and the oral dosage to patients, using either a newly developed rat model for acute hepatic damage by ethanol loading orally or using patients with the chronic type of alcoholic hepatic disorder. The enzymatic activities in the blood of rats and patients were monitored as parameters of the degree of the state of hepatic disorders.

We inventors determined that not only was the ethanol in blood rapidly cleared but also there was little possibility of eliciting histological changes in the liver similar to those in human alcoholic hepatitis when rats received an ethanol loading at the nearly lethal dose, previously reported. Also, attempts have been made to establish a chronic model for human hepatic disorders involving hepatitis induced by ethanol but there are very few reports of using rats successfully to produce a model sufficient to research for an etiological mechanism.

So we have tried to develop a new rat model for this disease and fortunately found out the acute model of alcoholic hepatitis in rats which received ethanol loading with a small amount of hydrazine sulfate. This model concerns the delayed ethanol clearance from the blood, the deterioration in detoxification and the elevation of the serum enzymatic activities originating from alcoholic hepatitis, such as glutamic oxaloacetic transaminase (GOT), glutamic puruvic transaminase (GPT) and ornithin carbamyl transferase (OCT).

Besides, this model became worse along with the dietary protein increase. So, we have succeeded in determining the optimal molar ratio of Ala and Gln to have a beneficial effect on the promotion of ethanol clearance from the blood as well as on the suppression of serum enzymatic activities elevated by alcoholic hepatitis, by pretreating rats with Ala and Gln in various ratios prior to ethanol loading with hydrazine sulfate.

Definitively speaking, the composition prepared according to the use of this invention is a composition, containing Ala and Gln, in which the molar ratio is preferably in the range from 1:0.1 to 1:10, the composition being applicable to the therapy of hepatic disorders, for example, alcoholic hepatic damage and its prevention. Both Ala and Gln should preferably be employed as the L-form enantiomers as natural products; however, no problems occurred whenever the ingredient contained any isomers, i.e., L-, D- and L- or D-form of both amino acids. Also Ala and Gln may be used as a free form, as salts or as peptide in residue.

The composition is preferably formulated with Ala and Gln in free form, with the total content in a range from 1g to 20g per capita (adults weighing from 40kg to 70kg) per day.

It is surely acceptable to be effective and beneficial whenever the ingredient contains both Ala and Gln in some molar ratio; therefore, it is also possible that each compound is separately prepared and both are concurrently ingested.

Treatment with a composition containing Gln alone or as major component of a mixture promoted ethanol clearance from the blood and diminished the depression including death after ethanol loading. On the other hand, treatment with composition containing Ala alone or predominantly is quite effective at suppressing the elevation of enzymatic activities in the serum of rats after hepatitis has been elicited by ethanol loading.

These data indicate that therapeutic effectiveness occurred in rats with alcoholic hepatic disorders, when the pharmaceutical composition was formulated with ala and Gln in a molar ratio ranging from 1:0,1 to 1:10, and that the optimal ratio of both compounds is 1:1. In addition, 3g of a composition comprising Ala and Gln (the molar ratio as 1:0.13), was tested in patients with the chronic type of alcoholic hepatic disorders, and the enzymatic activities in their serum of GOT, GPT and r-GTP, clearly declined, precisely supporting the data from the experimental models mentioned before. Regarding as little drugs to decrease the serum r-GTP activity, this invention is a worthwhile therapy for these disease. The effective dosage of this composition and the duration of therapy in patients to be recommended is not less than 1g up to 20g per capita(adults) per day for not less than one month. Of course, the dosage and the duration are changeable depending upon the state of disease. Besides, both Ala and Gln are also available for use as salts or analogues in the composition but any compounds included should be metabolized to the free forms of Ala and Gln in the living body.

Also the composition is useful as a drug as well as a food in case of the specification within the limits of molar ratio and dosage. It is recommended to use this ingredient for pharmaceutical use as follows: powdered, granulated, tablet-made, sugar-coated encapsulated or as a liquid drug. Additionally, beverages and chewing gums containing Ala and Gln should be beneficial to serve for people concurrently or pre- and post-ingestion with alcoholic beverages and liquors.

Indeed, the composition may be formulated with other amino acids and their analogues in addition to Ala and Gln within the specification involving sufficient amounts in the adequate molar ratio of Ala and Gln as indispensable compounds.

### Example I

Sprague-Dawley strain male rats, 10 weeks of age, body weight around 300 g, were used. Each animal was housed individually in a stainless steel case and fed a commercial laboratory chow ad libitum until 3 days prior to treatment. The animal room was illuminated from 7 a.m. for 12 h. The experimental diet containing 15% (w/w) whole egg protein (PEP) was offered to rats for 2 days, then a high protein diet with 50% PEP for 2 days. On the first day the high protein diet was offered, each animal received by intubation, Ala and/or Gln in suspension with 0.3% carboxymethyl cellulose (201 mg Nitrogen/kg BW) or vehicle (control) as a pretreatment (N = 10, each group). The ratio of both compounds in mixture was varied as follows; Ala or Gln alone, and both Ala and Gln in molar ratios of 1:0,2, 1:1, or 1:5.

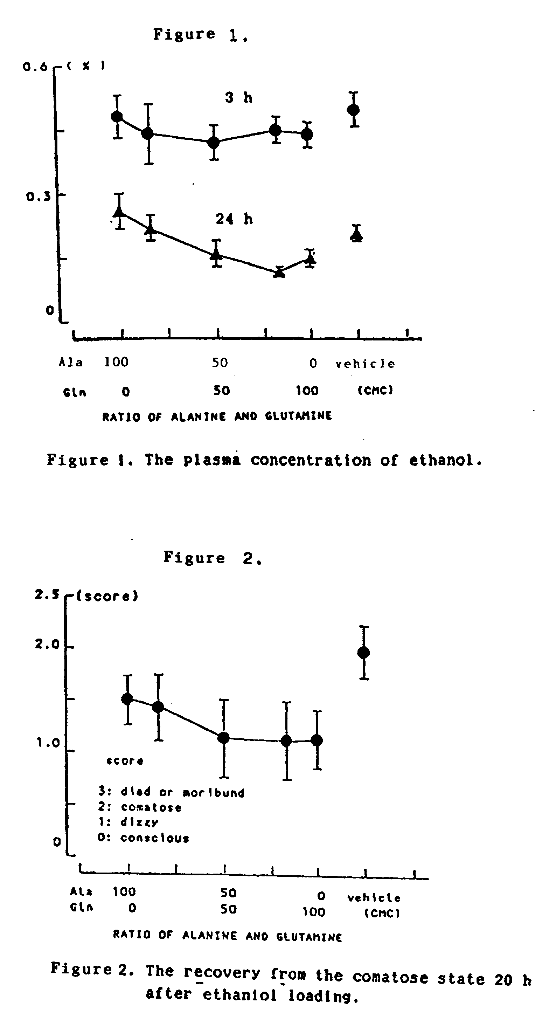

They were intubated again with 20% (w/v) ethanol in saline (6g/kg BW) with 0.13% (w/v) hydrazine sulfate (0.3 mmole/kg BW) 1h after the pretreatment. Blood collection from the infraclavicular vein in individuals of each group including control at 3 and 24 h after the treatment was performed. Clinical observation was also carried out at 3 and 20 h after treatment. The concentration of ethanol in plasma was assayed by the method of Bucher and Redetzki (Klin. Wochenschr 29: 615, 1951). The comatose state in individuals after ethanol loading orally were scored as follows; 0, dead or moribund; 1, comatose, 2; dizzy, and 3; conscious. Data from the present study were expressed as mean value with a standard error and are summarized in figures 1 and 2.

The concentration of ethanol in the plasma of the group receiving Gln alone after 3h of ethanol loading orally, was lowered significantly in comparison with controls. This effect was much more enhanced in any groups receiving a mixture of Ala and Gln, in a molar ratio not less than 1:1. This characteristic change became clearer at 24 h after ethanol treatment (figure 1). On the other hand, the comatose state in each group was essentially the same at 3h after the ethanol loading, but the recovery from this coma at 20 h following to treatment was greatly ameliorated by the intubation of both Ala and Gln as a pretreatment. This effect was enhanced when the Gln moiety was increased in the mixture, and reached a plateau level when the molar ratio (Ala:Gln) was less than a value of 1:1 (figure 2).

### Example II

Sprague-Dawley strain male rats, 10 weeks of age, body weight around 300 g, were used. Each animal was individually housed in a stainless steel case and fed a commercial laboratory chow ad libitum until 3 days prior to treatment. The animal room was illuminated from 7 a.m. for 12 h. The experimental diet containing 15% (w/w) PEP was offered to rats for 2 days, then a high protein diet with 50% PEP for 2 days. On the first day the high protein diet was offered, each animal received, by intubation, Ala and/or Gln in suspension with 0.3% carboxymethyl cellulose (201 mg Nitrogen/kg BW) or vehicle (control) as a pretreatment. The ratio of both compounds in mixture was varied as follows; Ala or Gln alone, and both Ala and Gln in molar as 1:0,2, 1:1 or 1:5 (N = 5, each group).

They were intubated again with 20% (w/v) ethanol in saline (7g/kg BW) with 0.11% (w/v) hydazine sulfate (0.3 mmole/kg BW) 1 h after the pretreatment. The clinical observation in individuals of each group including controls at 3, 24 and 72 h after the ethanol treatment was performed.

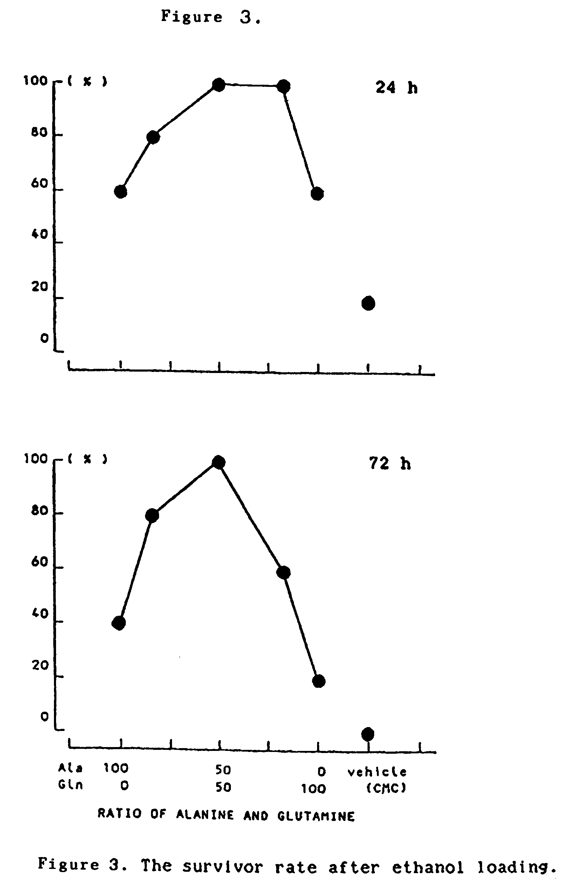

The survival rate of each group was recorded and is summarized in figure 3. Only one rat in five controls, which received intubation with vehicle as a pretreatment, was alive at 24 h and all animals were dead at 72 hr after ethanol loading. But the survival rate of any group which received the intubation with Ala and/or Gln, was better than that in controls and data were noted as follows; Ala alone, 3/5 deaths, Gln alone, 4/5 deaths, and both, the molar ratio (Ala:Gln) 1:0,2, 1/5; 1:1, 0/5; and 1:5, 2/5.

### Example III

Sprague-Dawley strain male rats, 10 weeks of age, body weight around 300 g₁ were used. Each animal was individually housed in a stainless steel cage and fed a commercial laboratory chow ad libitum until 3 days prior to treatment. The animal room was illuminated from 7 a.m. for 12 h. The experimental diet containing 15% (w/w) PEP was offered to rats for 2 days, then a high protein diet with 50% PEP for 2 days. On the first day of high protein diet offered, each animal received by intubation Ala and/or Gln in suspension with 0.3% (w/v) carboxymethyl cellulose (201 mg Nitrogen/kg BW) or vehicle (control) as a pretreatment (N = 10, each group). The ratio of both compounds in mixture was varied as follows; Ala or Gln alone, and both Ala and Gln in molar ratio as 1:0,2, 1:1, or 1:5.

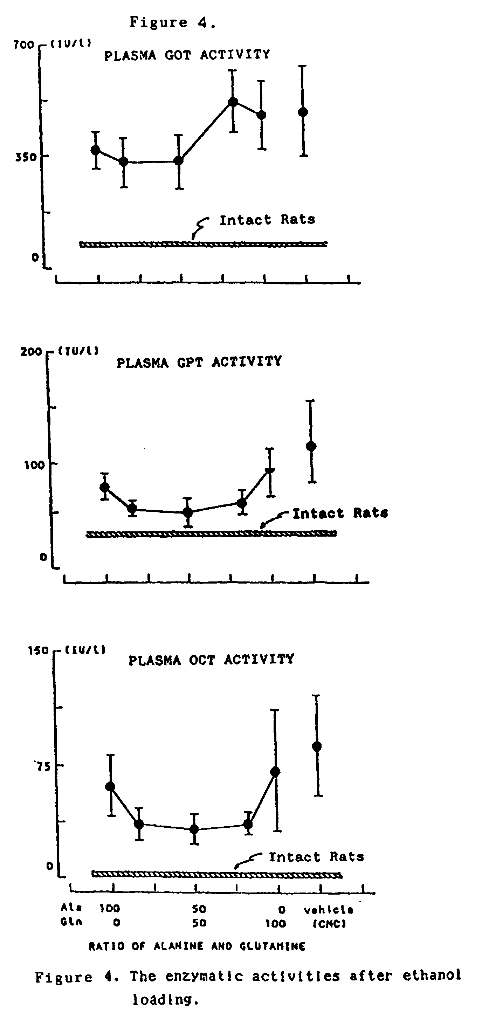

They were intubated again with 20% (w/v) ethanol in saline (6g/kg BW) with 0.13% (w/v) hydrazine sulfate (0.3 mmole/kg BW) 1 h after the pretreatment. Blood collection from the infraclavicular vein and clinical observation of individuals at 3 and 24h after the treatment was performed. The yielded plasma was assayed for enzymatic activities derived specifically from the liver following alcoholic hepatitis induced by the ethanol loading orally; GOT and GPT by the method of Henry R.J. et al. (Am. J. Clin, Path. 34: 381, 1960) and OCT by the method of Oshita, M. et al. (Clin. Chim. Acta. 67: 145, 1976). Data from each experimental group are summarized in figure 4 and the mean value with a standard error for intact rats was noted in this figure. These enzymatic activities in plasma of controls were greatly enhanced by ethanol loading, suggesting that acute hepatitis occurred. But any pretreatment of Ala and/or Gln clearly suppressed the increments of these activities, and especially the combination of both compounds was shown to be more effective than either alone (figure 4.)

### EXAMPLE IV

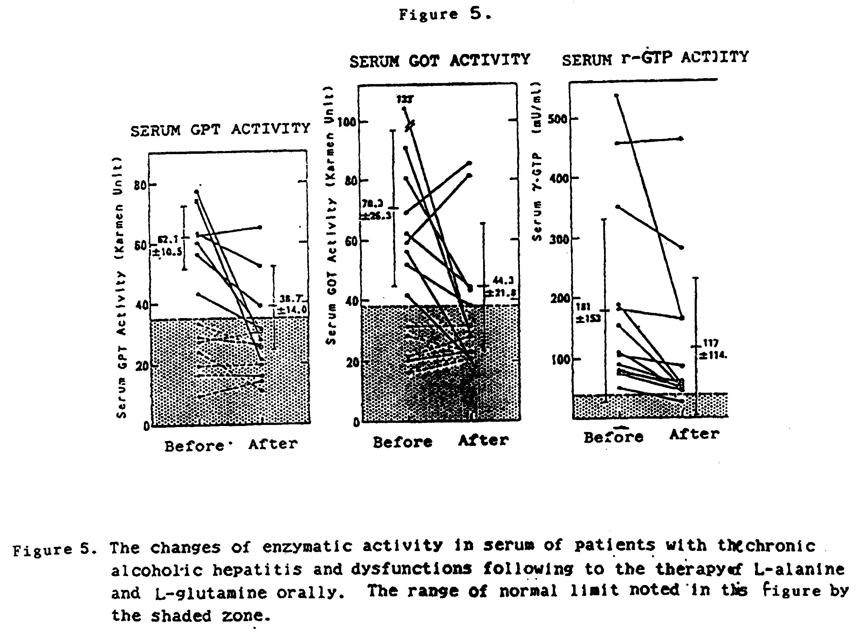

Fourteen patients with the alcoholic hepatitis, who were free from the hepatic virus, i.e. both types A and B, were selected from people, who have consumed large amounts of alcoholic beverages constantly for long term (more than 20 years) and been consulting their disease with our own hospital. They received the therapy of Ala and Gln in mixture orally (1g) three times until 30 min after meal (totally 3g per day) for prolonged period ranging from 1 to 6 months. The molar ratio of Ala and Gln was 1:0,12. The enzymatic activities of GOT, GPT or r-GTP in serum of patients, having been beyond the normal limits, became lower than the onset of this therapy and data was noted as follows: GPT, 6 in 7 cases, GOT, 7 in 9, r-GTP, 13 in 14 (figure 5).

Neither complaints nor any adverse effect related to this treatment were recorded. Also there were no cases where the prescription and protocol of this therapy were not observed, but almost all patients still consumed alcoholic beverages despite of abstinence from drink strongly suggested. These data indicate that the therapy of Ala and Gln in mixture orally could be effective to amerliorate the state of alcoholic hepatitis.

Regarding as these evidences having been mentioned in this petition, it become clear that the mixture composing of Ala and Gln within the specification is practically useful either as a clinical drug for the treatment of hepatic dysfunction including alcoholic hepatitis or to prevent this disease in people whose habit is to drink a lot. This discovery is quite valuable as pharmaceutical use.

### Figure Legends

The plasma concentration of ethanol and the comatose state of ethanol treated rats, which received intubation of Ala and/or Gln as a pretreatment (Example I) are shown in figures 1 and 2. The survival rate (Example II) and the enzymatic activities in serum of rats (Example III) and patients with alcoholic hepatitis (Example IV) were also shown in figure 3, 4 and 5.

Figure 1 shows the plasma concentration of ethanol.
a) 3 h after ethanol loading (upper).
b) 24 h after ethanol loading (bottom).

Controls were intubated with vehicle (0.3% carboxymethyl cellulose in saline, abbreviated as CMC.).

Figure 2 shows the recovery from the comatose state 20 h after ethanol loading.

The degree of recovery from the comatose state in each rat was scored as follows; 3), dead or moribund, 2)., still comatose, 1)., dizzy and depressed and 0)., conscious and completely recovered. Controls were intubated with vehicle (0.3% CMC in saline).

Figure 3 shows the survival rate after ethanol loading.
a) 24 h after treatment (upper)
b) 72 h after treatment (bottom)

Controls were intubated with vehicle (0.3% CMC in saline).

Figure 4 shows the enzymatic activities after ethanol loading.
A). Plasma GOT activity (upper).
B). Plasma GPT activity (center).
C). Plasma OCT activity (bottom).

Controls were intubated with vehicle (0.3% CMC in saline). Mean values with a standard error from intact rats without any treatment were noted in each figure.

Figure 5. The changes of enzymatic activity in serum of patients with the chronic alcoholic hepatitis and dysfunctions following therapy with L-alanine and L-glutamine orally.
A). Serum GPT activity (left).
B). Serum GOT activity (middle).
C). Serum r-GTP activity (right).

The range of normal limit is noted in this figure by the shaded zone. The changes of enzymatic activity beyond normal limit
(⊷)
or within normal limit
( 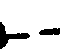 )
were separatedly expressed in each figure. Data from patients with the enzymatic activity beyond normal limit before and after the therapy of Ala and Gln were noted in each figure as the mean value with a standard error.

## Claims

1. Use of L-alanine or a metabolic equivalent thereof and L-glutamine or a metabolic equivalent thereof in the manufacture of a pharmaceutical composition for the treatment or prophylaxis of an alcoholic hepatic disorder.

2. Use according to Claim 1 employing a said equivalent of L-alanine and/or of L-glutamine which is a salt, derivative or peptide.

3. Use according to Claim 1 or Claim 2 wherein the composition is a two-component composition, one component containing said L-alanine or equivalent thereof, the second component containing said L-glutamine or equivalent thereof.

4. Use according to any one of the preceding claims wherein the composition comprises L-alanine or a metabolic equivalent thereof and L-glutamine or a metabolic equivalent thereof in molar ratios of from 1: 0.1 to 1:10.

5. Use according to Claim 4 wherein the molar ratio of L-alanine or a metabolic equivalent thereof: L-glutamine or a metabolic equivalent thereof in the composition is about 1:1.

6. Use according to any one of the preceding claims wherein the L-alanine is accompanied by D-alanine and/or the L-glutamine is accompanied by D-glutamine.

7. Use according to any one of the preceding claims wherein the composition further comprises one or more pharmaceutically acceptable diluents or carriers to form a medicament which is suitable for oral administration.

8. Use according to Claim 7 wherein the composition takes the form of a powder, granule, tablet, capsule, a liquid or chewing gum.

9. Use according to Claim 7 or Claim 8 wherein the composition is suitable to provide a dosage of 1 to 20 g/person/day.

## Patentansprüche

1. Verwendung von L-Alanin oder eines metabolischen Äquivalents davon und L-Glutamin oder eines metabolischen Äquivalents davon für die Herstellung einer Arzneimittelzusammensetzung zur Behandlung oder Prophylaxe von alkoholbedingten Leberfunktionsstörungen.

2. Verwendung gemäß Anspruch 1, bei der ein Äquivalent von L-Alanin und/oder L-Glutamin eingesetzt wird, welches ein Salz, Derivat oder Peptid ist.

3. Verwendung gemäß Anspruch 1 oder Anspruch 2, bei der die Zusammensetzung eine Zweikomponenten-Zusammensetzung ist, wobei eine Komponente das L-Alanin oder dessen Äquivalent und die zweite Komponente das L-Glutamin oder dessen Äquivalent enthält.

4. Verwendung gemäß einem der vorhergehenden Ansprüche, bei der die Zusammensetzung L-Alanin oder ein metabolisches Äquivalent davon und L-Glutamin oder ein metabolisches Äquivalent davon in Molverhältnissen von 1:0,1 bis 1:10 enthält.

5. Verwendung gemäß Anspruch 4, bei der das Molverhältnis von L-Alanin oder eines metabolischen Äquivalents davon zu L-Glutamin oder eines metabolischen Äquivalents davon in der Zusammensetzung etwa 1:1 beträgt.

6. Verwendung gemäß einem der vorhergehenden Ansprüche, bei der das L-Alanin von D-Alanin und/oder das L-Glutamin von D-Glutamin begleitet ist.

7. Verwendung gemäß einem der vorhergehenden Ansprüche, bei der die Zusammensetzung zusätzlich ein oder mehrere pharmazeutisch geeignete Verdünnungsmittel oder Trägermaterialien enthält, so daß ein für die orale Verabreichung geeignetes Arzneimittel gebildet wird.

8. Verwendung gemäß Anspruch 7, bei der die Zusammensetzung die Form eines Pulvers, Granulats, einer Tablette, Kapsel, einer Flüssigkeit oder von Kaugummi hat.

9. Verwendung gemäß Anspruch 7 oder Anspruch 8, bei der die Zusammensetzung geeignet ist, um eine Dosis von 1 bis 20 g/Person/Tag zur Verfügung zu stellen.

## Revendications

1. Utilisation de L-alanine ou d'un équivalent métabolique de cette dernière et de L-glutamine ou d'un équivalent métabolique de cette dernière pour la préparation d'une composition pharmaceutique destinée au traitement ou à la prévention de troubles hépatiques d'origine éthylique.

2. Utilisation selon la revendication 1, employant un dit équivalent de la L-alanine et/ou de la L-glutamine qui est un sel, un dérivé ou un peptide.

3. Utilisation selon la revendication 1 ou la revendication 2, dans laquelle la composition est une composition à deux composants, un composant contenant ladite L-alanine ou son équivalent, le second composant contenant ladite L-glutamine ou son équivalent.

4. Utilisation selon l'une quelconque des revendications précédentes, dans laquelle la composition comprend de la L-alanine ou un équivalent métabolique de cette dernière et de la L-glutamine ou un équivalent métabolique de cette dernière dans des rapports molaires compris entre 1:0,1 et 1:10.

5. Utilisation selon la revendication 4, dans laquelle le rapport molaire de la L-alanine ou d'un équivalent métabolique de cette dernière à la L-glutamine ou un équivalent métabolique de cette dernière dans la composition est d'environ 1:1.

6. Utilisation selon l'une quelconque des revendications précédentes, dans laquelle la L-alanine est accompagnée de D-alanine et/ou la L-glutamine est accompagnée de D-glutamine.

7. Utilisation selon l'une quelconque des revendications précédentes, dans laquelle la composition comprend en outre un ou plusieurs diluants ou véhicules pharmaceutiquement acceptables pour former un médicament convenant à l'administration orale.

8. Utilisation selon la revendication 7, dans laquelle la composition prend la forme d'une poudre, de granules, de comprimés, de capsules, d'un liquide ou d'une gomme à mâcher.

9. Utilisation selon la revendication 7 ou la revendication 8, dans laquelle la composition est utilisable pour fournir une posologie de 1 à 20 g/personne/jour.
